# EUROPEAN PATENT SPECIFICATION

(11) **EP 1 033 118 B1**
(45) Date of publication and mention of the grant of the patent: **20.07.2005**
(21) Application number: 00103868.6
(22) Date of filing: 24.02.2000
(51) Int. Cl.: A61F 5/08

(54) **Nasal dilator**
Nasaler Dilatator
Dilatateur nasal

(30) Priority: 02.03.1999 US 260445
(43) Date of publication of application: 06.09.2000
(73) Proprietor: 3M Innovative Properties Company, St. Paul, Minnesota 55133-3427 (US)
(72) Inventor: Delmore, Michael D., Saint Paul, Minnesota 55133-3427 (US); Pendergrass, Daniel B., Jr., Saint Paul, Minnesota 55133-3427 (US); Neeser, Roger D., Saint Paul, Minnesota 55133-3427 (US); Mallo, Richard A., Saint Paul, Minnesota 55133-3427 (US)
(74) Representative: Hilleringmann, Jochen, Dipl.-Ing.

(56) References cited:
- EP-A- 0 195 254
- WO-A-98/27897
- WO-A-98/57613
- US-A- 5 455 043
- US-A- 5 706 800

## Description

This invention relates to medicated nasal dilators. More specifically, this invention relates to nasal dilators comprising microencapsulated volatile medications.

### Background of the Invention

Nasal dilators are devices used to open up nasal passages to increase airflow through the nasal passage. Nasal dilators can be applied to the exterior surface of the nose. A biasing force from a spring element or the like exerts an outward force on the outer tissue wall opening of the nasal passages. These devices can be used for medical related problems such as deviated septa, allergic swelling, or the like. These devices are also used to alleviate snoring, for increased athletic performance, and like uses.

US-A-5,706,800 describes nasal dilators and methods for improving the breathing of individuals, wherein an aromatic medication or transdermal medication is disposed on the dilator to further improve breathing.

WO-A-98/57613 discloses an adhesive plaster which can be used as a nasal dilator, comprising a backing substrate whose first face is provided with an adhesive layer and second face with microcapsules containing volatile medication.

Medicated nasal dilators of the prior art suffer from disadvantages because the medication provided on the device is subject to dissipation over time on the shelf. To avoid such dissipation, the medicated nasal dilator could conceivably be provided in hermetically sealed packaging, but such packaging is very expensive. Further, it is difficult to manufacture truly hermetically sealed containers for volatile medications, because such medications may be capable of escaping from packages that appear to be sealed. For example, many volatile materials may escape by migration through many packaging adhesives. Yet another alternative of the prior art is to have users apply the medication to the nasal dilator themselves, which requires time and energy from the user and also may be messy.

### Summary of the Invention

The present invention is directed to a nasal dilator as defined in claim 1. The dependent claims relate to individual embodiments of the invention.

The present invention is a solution to the problem of providing medication with a nasal dilator in an easy to use and shelf stable manner. Nasal dilators of the present invention are devices that are applied by a pressure-sensitive adhesive to the nasal skin of the tissue forming the outer side wall of the nasal passages. The devices incorporate a biasing force (for example, from a spring element or the like) that exerts an outward force on the outer tissue wall opening of the nasal passages, thereby opening the nasal passage for easier breathing. Examples of constructions of these nasal dilators are described in U.S.-A-5,549,103; US-A-4,414,977; US-A-1,292,083; US-A-1,950,839; US-A-5,476,091; US-A-5,533,499; US-A-5,533,503; US-A-5,546,929; US-A-5,553,605; US-A-5,611,333; US-A-5,611,334; US-A-5,653,224; US-A-5,706,800; US-A-5,718,224; WO-A-94/23675; WO-A-97/02798; WO-A-98/25541; EP-A-0 855 175 and US-A-5,957,126. As noted above, the present invention is directed to a nasal dilator comprising a backing substrate having a first face and a second face. The first face is provided with an adhesive layer and the second face provided with microcapsules containing volatile medication.

Providing the volatile medication both in encapsulated form and with the microcapsules located on the second face of the backing substrate additionally protects the adhesive of the nasal dilator from contamination with the medication. Such contamination could adversely affect the adhesive properties of the dilator, or adversely affect the medicative properties of the medication. Preferably, the microcapsules are located on the spring elements, which are generally made of liquid impervious materials and therefore provide an excellent barrier to contamination of the adhesive on the first face of the nasal dilator by the contents of the microcapsule after microcapsule rupture.

According to the present invention the microcapsules are provided under a liquid or vapor permeable protective layer. This embodiment allows the use of large microcapsules, which have a very large payload of medication, but which also could exhibit a disadvantage in shedding relatively large and unsightly fragments of microcapsule shells from the device. The overlayer of liquid permeable projective layer thus retains these unsightly shell fragments within the device, while allowing release of the volatile liquid medication.

The liquid or vapor permeable protective layer may be paper, polymeric film, coated paper, polymeric foam, elastomer, a nonwoven sheet or the like. Paper, polymer film and coated paper are preferred. For good results in this embodiment, the microcapsules should have an average diameter in the range of 200-3000 microns. Microcapsules more than 3000 microns in diameter are less desired because to make them handleable in manufacturing and processing they must have rather thick, strong shells that hinder their easy rupture in the nasal dilator. At sizes smaller than 200 microns, the microcapsules become rather difficult to rupture by hand pressure. Preferably the microcapsules are in a range of about 300-1000 microns in diameter.

In one embodiment of the present invention, microcapsules are provided on the second face of the backing substrate in a manner such that the medication is released by rubbing the face of the substrate, thereby rupturing the microcapsules. Alternatively, the nasal dilator may be exposed to microcapsule rupturing force, either before or after removal of the nasal dilator from individual packaging that is a preferred delivery format of such devices. For example, the user may rub, roll or otherwise impart force to the package containing the nasal dilator with the hand or a hard object in order to rupture the microcapsules on the second face of the backing substrate. This embodiment has a particular advantage in that the user may select whether to release volatile medication or not by rupturing or not rupturing the microcapsules. Further, it is typically observed that only a portion of the microcapsules are ruptured in the first release of volatile medication. This embodiment therefor provides the ability for the user to refresh the release of volatile medication on demand by rubbing the nasal dilator while it is in place on the nose. The user may also control the relative amount of release of volatile medication by rupturing more or fewer microcapsules through, for example, rubbing more or less vigorously.

Preferably, the microcapsules of this embodiment are attached to the second face of the backing substrate by a composition that is of the class of materials called Low Adhesion Backsize ("LAB"). These materials have relatively low surface energy as compared to traditional binders, and when coated on the intended substrate do not stiffen the substrate and also provide a non-sticky surface. The resulting coating comprising microcapsules is hydrophobic, so that the top portion of the nasal dilator is moisture resistant. Moisture resistance is a particular advantage in this invention, because it is undesirable for nasal dilators to absorb sweat or other fluids that would make the nasal dilator uncomfortable or unsuitable for use. The term "Low Adhesion Backsize" refers to a material which readily releases from a layer of pressure sensitive adhesive and includes, but is not limited to, silicones, fluorochemicals, acrylates, urethanes, polyethylenes, chrome complexes, grafted or block siloxane hydrocarbons, vinyl copolymers, and blends of these materials. Examples of various low adhesion backsizes are found in U.S.-A-4,421,904; US-A-4,313,988; US-A-4,279,717; U.S.-A-4,728,571. Other low adhesion backsizes which may be used according to the present invention are described in U.S.-A-2,607,711, US-A-4,973,513, US-A-2,876,894; and US-A-2,532,011. A specific example of a suitable low adhesion backsize is SYL-OFFä, a silicone compound available from Dow Corning Corp. Preferred low adhesion backsizes are the siloxane and acrylate based compounds disclosed in U.S.-A-4,973,513 and the water-insoluble hydrophobic urethane (carbamate) copolymer of polyvinyl alcohol and octadecyl isocyanate disclosed in U.S.-A-2,532,011.

In another embodiment, the second face is adhesively bonded to a cover sheet with an adhesive composition containing the microcapsules. The cohesive strength of the adhesive composition is less than the strength of the bond between the adhesive composition and the second face and the cover sheet. The tensile rupture strength of the microcapsules is such that the cohesive failure of the adhesive results in the breakage of the microcapsules. In a preferred aspect of this embodiment, the cover sheet is part of a package for the nasal dilator. Thus, when the package is taken apart, the dilator is peeled away from the top sheet of the package and the microcapsules on the second face of the dilator are ruptured during this peeling process. These embodiments of the present invention further provide storage stability benefit, because the cover sheet may provide an occlusion effect, whereby microcapsules that are otherwise not completely sealed (i.e. "leaky") are physically blocked from releasing the volatile medicament.

In yet another embodiment of the present invention, the microcapsules are provided in a binder matrix that is stiff, such that upon application of force such as tension or shear force to the nasal dilator, the microcapsules are ruptured. Preferably, the force applied to the nasal dilator that results in the rupture of microcapsules is that of flexing of the nasal dilator. Most preferably, the force applied to the nasal dilator is only the amount of flexing required to conform an originally generally planar nasal dilator to the generally arched configuration over the bridge of the nose. Advantageously, microcapsules may be located on the spring elements in this embodiment in order to minimize flexing of the microcapsule containing binder composition during manufacture, since the spring element will minimize flex of the nasal dilator in the converting process.

The binder matrix of this embodiment of the present invention is preferably a polymeric material having a glass transition temperature (T_{g}) such that the total binder matrix is sufficiently stiff at ambient temperature of application of the nasal dilator to the nose, so that microcapsules are ruptured upon flexing of the nasal dilator in application to the nose. Preferably, the (T_{g}) of the polymeric material comprising the majority of the matrix is above about 49° C (120° F), so that microcapsules are ruptured upon flexing of the nasal dilator in application to the nose at an ambient temperature that would generally be below about 49°C (120° F). A particularly preferred binder matrix comprises polymer and other materials such that the matrix is stiff at temperatures below about 49° C (120° F) so that microcapsules are ruptured upon flexing of the nasal dilator in application to the nose, yet is sufficiently flexible at temperatures above about 60° C (140° F) so that the device may be manufactured at an elevated temperature without substantial microcapsule rupture.

Binders useable in this embodiment of the invention include emulsion polymerized acrylates, solution polymerized acrylates, and tackified, anionically polymerized ABA block copolymers, isocyanate terminated prepolymers, and epoxy resins. Other common binders, e.g., urethanes, polyethers, and polyesters, could also be used. The major variables which can be tailored for each application are the desired T_{g} of the polymer to select the temperature range at which microcapsule rupture becomes significant, the ability of the binder to adhere to the substrate, and the ability of the binder to adhere to the microcapsules so that stress may be concentrated at the microcapsule wall. These characteristics may be achieved by selection of polymers either individually or by blending various polymers to formulate an effective T_{g}, for the overall binder matrix and to achieve desired adhesiveness based on dispersion of functional groups on the polymer throughout the matrix. The binder matrix may also be rendered more sensitive to strain through incorporation of fillers. As a general rule, the more non-elastic filler that is present in the binder matrix, the more localized a strain is on the polymeric portion of the binder matrix. A highly filled matrix therefore tends to be more sensitive to strain. These parameters can be tailored for a given application by one of ordinary skill in the art with only routine experimentation. The binder for the microcapsules may be crosslinked after assembly to adjust the stiffening temperature upwards.

The binder of this embodiment may further be selected so that it has a relatively low initial stiffening temperature so that initial manipulations may be performed on the nasal dilator at ambient temperature without concern of rupturing the microcapsules. The stiffening temperature of the binder may optionally be adjusted during manufacture after any converting steps requiring flexing of the nasal dilator. This post-converting stiffening operation may even take place after placement of the nasal dilator in the final package. For example, the polymer in the binder may be crosslinked or may undergo a chain extension reaction by exposure to radiative cure (such as visible or UV light cure or E-beam cure), heat or chemical cure (such as exposure of reactive polymeric binders, such as epoxy resins and isocyanate terminated prepolymers, to appropriate amine vapors or the like). Alternatively, the binder matrix may be cured by aerobic or anaerobic cure mechanisms or other means of stiffening, such as loss of solvent (including materials that are traditionally referred to as plasticizers).

In another embodiment of the present invention, the microcapsules can be provided on the cover sheet, with a liquid or vapor permeable protective layer adhered to the cover sheet. In this embodiment, the microcapsules are ruptured before removal of the cover sheet, and the volatile medication passes through the liquid or vapor permeable protective layer and resides on the second face of the backing substrate. In such embodiments, the user has the option of having a nasal dilator that delivers volatile medication or not delivering volatile medication by choosing whether to rupture the microcapsules. Optionally, the protective sheet in this embodiment may be adhesive to the second face of the backing substrate so that the nasal dilator is secured within the package and maintains good contact between the second face and the protective sheet.

In accordance with the present invention, microcapsules may be prepared by any appropriate microcapsule formation technique. A preferred technique is an in situ process such as aminoplast polymerization. The techniques disclosed, generally referred to as an in situ polymerization reaction, yield for example, an aminoplast resin microcapsule wall material. In the process, a hydrophobic oil phase is dispersed in an aqueous phase containing the aminoplast resin precursors by applying high shear agitation. Addition of an acid catalyst initiates the polycondensation of the aminoplast precursors, resulting in the deposition of the aminoplast resin about the dispersed droplets of the oil phase, producing the microcapsules. The hydrophobic inner phase for the microcapsule may be any in situ aminoplast encapsulatable composition as discussed in U.S. Pat. No. 3,516,941.

When the microcapsule is prepared by interfacial polycondensation, the microcapsule skin may be composed of any condensation polymer or addition polymer, e.g., polyamide, polyurethane, polysulfonamide, polyurea, polyester, polycarbonate, etc. Polyamides prepared by interfacial polycondensation of an amine with an acid chloride or polymers formed by reaction of isocyanate prepolymer with polyamines are preferred. Microcapsules formed by coacervation processes are also useful in forming microcapsule shells according to the present invention. Coacervation is the well-known process of forming higher molecular weight gelatin polymers as taught in U.S.-A-5,800,458 and US-A-2,800,457. It should be noted that microcapsules made according to the two techniques described in this paragraph are generally somewhat leaky, and may lead to early release of volatile medications, particularly those that comprise very small molecules prone to escape from such materials.

The microcapsules used in these constructions and generally in the practice of the present invention generally have average diameters between about 4 and 200 microns, although larger capsules are appropriate for certain embodiments, particularly as noted above. Preferably the average diameters are between about 40 and 150 microns, and more preferably, between about 80-120 microns. The microcapsules preferably constitute from 2 to 55% by weight of the volatile medication, and most preferably between 5 and 40% by weight of the volatile medication. Larger microcapsules deliver much more volume of volatile medication per microcapsule, and therefore are preferred for the present invention.

Medications according to the present invention are encapsulatable volatile materials that provide benefit to the user when inhaled. The medications are considered to be volatile if when released to the surface of a nasal dilator from rupture of microcapsules, the medication vaporizes sufficiently to be inhaled by the patient in an amount effective to provide benefit to the patient. Examples of such medications include camphor, eucalyptus oil, peppermint oil, menthol, methyl salicylate, bornyl acetate, lavender oil, or a combination of these. Transdermal decongestants and antihistamines are also contemplated, such as diphenhydramine and triprolidine transdermal antihistamine, available from Proctor and Gamble Co., Inc., Cincinnati, Ohio. Other medications include ephedrine, dimethindene, epinastine, emedastine, and clonidine. Medications that are not regulated by the US FDA are also contemplated, including aromatherapy agents and natural therapy agents such as vanilla. The volatile medications may be provided in the microcapsules in undiluted form, or may preferably be diluted with an appropriate carrier solvent. Blends of volatile medications in the capsules are contemplated, either with or without a carrier solvent. Medications that are not particularly volatile may be rendered more volatile by mixing with a "lifter" solvent, which is a highly volatile solvent that will act to carry the less volatile medicament into the air. Multiple volatile medications may be provided in a single nasal dilator by encapsulation together in microcapsules containing both volatile medications, or by mixing microcapsules having only one volatile medication encapsulated therein with microcapsules having a different volatile medication encapsulated therein. In the case where microcapsules having different volatile medications in separate microcapsules are blended, the relative amounts of volatile medications may be easily metered in this way.

### Brief Description of the Drawings

The invention will be described in detail referring to the drawing in which:
Fig. 1 is an exploded perspective view of a nasal dilator, and
Fig. 2 is an exploded perspective view of an embodiment of a nasal dilator in accordance with the present invention.

### Detailed Description

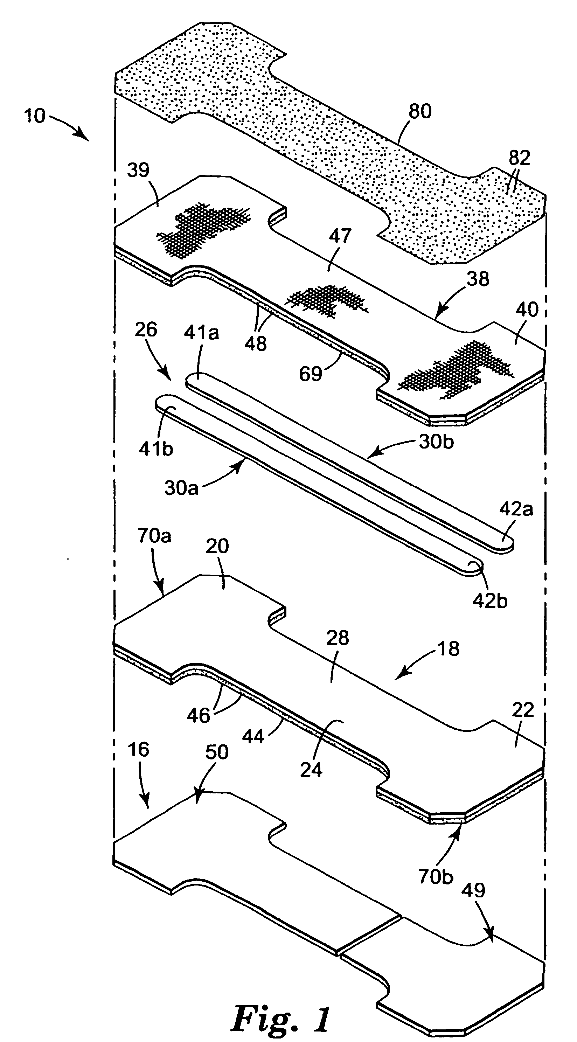

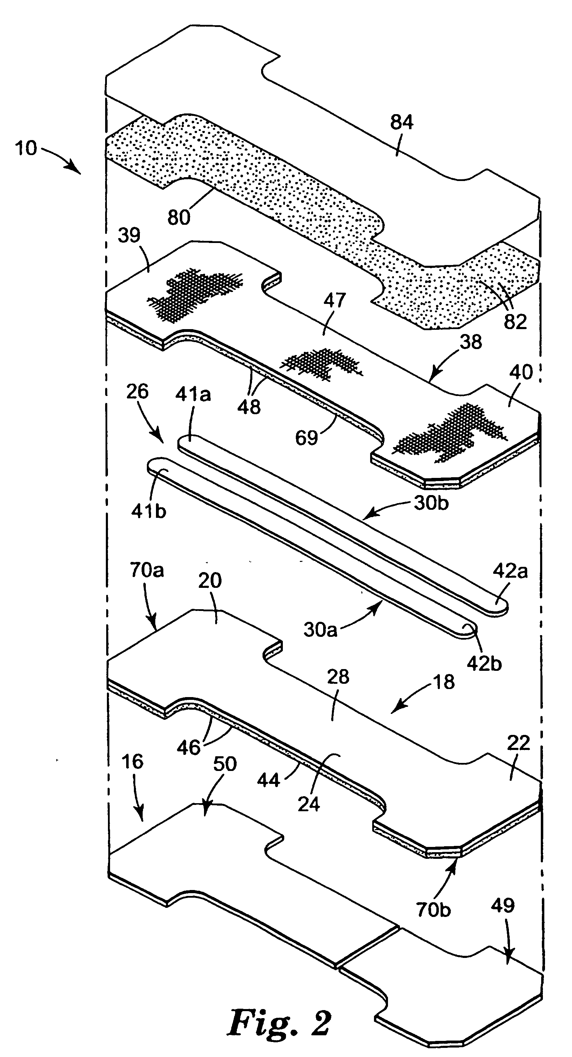

Turning now to the Drawing, an embodiment of the inventive nasal dilator 10 is depicted generally in Fig 2 and is used in the manner described in U.S.-A-5,549,103 and will be described with reference to Fig.1 first. The nasal dilator is applied as a pressure-sensitive adhesive article attached to the nose of the wearer at least at two end regions 20 and 22 of the backing 18. The invention adhesive layer 46 on at least these two ends 20 and 22 of a first face 44 is a pressure-sensitive adhesive layer 46.

The nasal dilator includes a resistant spring 26, which preferably includes one and preferably two or more resilient spring elements 30a and 30b. These resilient spring elements operate similar to a leaf spring providing a recovery force when bent around the nose of from 5 to 50 grams, preferably 20 to 30 grams force. Each element 30a and 30b is a portion of the total recovery force. These resilient spring elements can be formed of any suitable resilient material including metal, thermoplastic films, consolidated nonwovens, resin impregnated nonwovens or the like. A preferred material is an oriented polyester film stay of a thickness of from 200 microns (8 mils) to 375 microns (15 mils) preferably about 325 microns (13 mils). This stay is generally from 2 to 4 mm wide and comes in various lengths depending on the intended wearer, generally from 40 to 80 mm. The resilient spring elements 30 have first end regions 41a and 41b and second end regions 42a and 42b which end regions extend into first end regions 20 and second end region 22, respectively. The resilient element end regions generally extend into at least 50 percent or more of the attached end regions 20 and 22 of the backing 18, preferably at least 75 percent or more. The resilient spring elements 30a and 30b preferably extend to within 500 microns or less of the first and second ends 70a and 70b of the backing 18.

The backing 18 is preferably a breathable material such as a nonwoven web, a perforated film or a microporous film. The intermediate segment 24 generally has a minimum width less than the maximum width of the two end regions 20 and 22. Generally, the backing is attached to the nose along its entire length. However, the two end regions 20 and 22 can be attached to the nose such as by adhesive bonding while intermediate section 24 is free of adhesive contact with the nose. The enlarged end regions 20 and 22 allow for more secure contact with the nose in the area where the maximum forces are exerted by the resilient spring 26, for better distribution of force exerted by the resilient spring 26 and increased overall wearer comfort.

The resilient spring elements 30a and 30b are generally attached to the backing 18 by adhesive but other means of attachment could also be used, such as thermal bonding, sonic welding, physical entrapment (tube), or the like. Generally, the resilient spring elements are attached to the backing 18 along their entire length. However, the resilient spring elements 30a and 30b can be attached only to the end regions 20 and 22 of the backing 18 such as by patterned adhesive bonding, or use of a masking element in the intermediate segment 24. As shown in Fig. 1, the dilator 10 also preferably includes a top facing layer 38 which can be a breathable material as backing layer 18, or other suitable thin conformable material such as a nonwoven web such as a spunbond or meltblown web, a thin thermoplastic film such as a porous or perforated polyethylene film, on a woven or knitted material. This top facing layer is attached to the backing layer by adhesive lamination or the like and could also be attached to the resilient spring elements 30a and 30b. A suitable method of attachment is by use of an adhesive layer 48 on a bottom surface 69, which could be a pressure-sensitive adhesive or a hot melt adhesive. The top facing layer generally has a first end region 39, a second end region 40, and an intermediate section 47 which may be mirror images of the backing layer first end region 20, second end region 22, and intermediate section 24, respectively. However, the top facing layer can be attached primarily to the resilient elements 30a and 30b and extend to a minor degree to cover the backing layer around the periphery of the resilient spring elements. The top facing layer is primarily used to keep the resilient member attached to the backing layer, for aesthetics and comfort and to provide a more structurally integral laminate.

The adhesive layer 48 preferably is protected prior to use by one or more conventional release liners. There can be provided two release liners 49 and 50.

Microcapsule containing layer 80 containing microcapsules 82 in a binder is coated on top facing layer 47. Microcapsules 82 can be ruptured, for example, by scratching by the user, or other appropriate mechanism described above.

Fig. 2 shows the embodiment of the invention wherein like parts are identified by like reference numerals. Microcapsule-containing adhesive layer 80 is protected by cover sheet 84, which is adhered thereto by the adhesive of microcapsule-containing adhesive layer 80. The cohesive strength of the adhesive of microcapsule-containing adhesive layer 80 is less than the strength of the bond between the microcapsule-containing adhesive layer 80 and top face 47 and cover sheet 84, and the tensile rupture strength of microcapsules 82 is such that the cohesive failure of the adhesive of microcapsule-containing adhesive layer 80 results in the breakage of microcapsules 82.

The following examples are provided for purposes of illustrating the present invention, and are not intended to be limiting of the broadest concepts of the present invention. Unless otherwise indicated, all parts and percentages are by weight and all molecular weights are weight average molecular weight.

### EXAMPLE 1

### Nasal dilator with encapsulated menthol

The process described in US-A-3,516,941, was used to make urea formaldehyde microcapsules except menthol was the capsule pill material. Three parts urea formaldehyde microcapsules containing menthol were mixed with 20 parts urethane low adhesion backsize prepared as described by US-A-2,532,011. The mixture was agitated in a laboratory Silverson mixer commercially available from Silverson Co., Waterside, Chesham, Bucks, England for 2 to 3 minutes. The mixture was placed in a 1-liter (32-ounce) glass jar. The jar was sealed with a lid and placed on a roller for about 5 minutes. The mixture must be agitated until it is coated. The mixture was coated onto the nonadhesive surface of a 3M 1533L tape, 0.127 mm (5 mil) tan micropore rayon nonwoven adhesive coated on one side with a breathable, acrylic pressure-sensitive adhesive available from 3M Company, St. Paul, MN. A 30cm (twelve inch) wide gravure laboratory coater having an eighty rounds per minute (RM), eighty line, gravure cylinder at a coating speed of approximately 1.6 m per minute (5 feet per minute) was used to coat the tape. The tape was dried in a 66° C (150° F) oven with residence time of approximately 1 minute.

The microcapsule coated tape cover is secured to the resilient members of the nasal dilator as described in US-A-5,549,103.

## Claims

1. A nasal dilator (10) comprising
- a backing substrate (18) having a first face (44) and a second face, the first face (44) provided with an adhesive layer (46) and the second face (44) provided with microcapsules (82) containing volatile medication,
**characterized in that**
- the backing substrate (18) comprises at least one resilient spring element (30a,30b) and
- a liquid or vapor permeable protective layer (84) is provided over said microcapsules (82).

2. The nasal dilator (10) of claim 1, wherein the microcapsules (82) have a rupture strength and are dispersed in an adhesive composition, the composition having a cohesive strength and a first bond strength between it and the second face of the backing (18).

3. The nasal dilator (10) of claim 1 or 2, wherein the microcapsules (82) are bound to the second face by a binder material.

4. The nasal dilator (10) of claim 3, wherein the binder is a low adhesion backsize.

5. The nasal dilator (10) according to any one of claims 1 to 4, wherein the binder matrix is sufficiently stiff at about 49 °C (120 °F) so that microcapsules (82) are ruptured upon flexing of the nasal dilator (10) in application to the nose.

6. The nasal dilator (10) of claim 5, wherein the binder matrix is sufficiently flexible at temperatures at about 60 °C (140 °F) so that the nasal dilator (10) may be manufactured at an elevated temperature without substantial microcapsule rupture.

7. The nasal dilator (10) of any one of claims 1 to 6, wherein the microcapsules (82) have average diameters between about 4 and 200 microns.

8. The nasal dilator (10) of any one of claims 1 to 6, wherein the microcapsules have average diameters between about 300-1000 microns.

9. The nasal dilator (10) of any one of claims 1 to 8, wherein the volatile medication is selected from the group consisting of camphor, eucalyptus oil, peppermint oil, menthol, methyl salicylate, bornyl acetate, lavender oil, or mixtures thereof.

10. The nasal dilator (10) of any one of claims 1 to 8, wherein the volatile medication is selected from the group consisting of transdermal decongestants and antihistamines.

11. The nasal dilator (10) of any one of claims 1 to 8, wherein the volatile medication is selected from the group consisting of diphenhydramine, triprolidine, ephedrine, dimethindene, epinastine, emedastine, and clonidine.

12. The nasal dilator (10) of any one of claims 1 to 8, wherein the volatile medication is selected from the group consisting of aromatherapy agents and natural therapy agents.

13. The nasal dilator (10) of any one of claims 1 to 12, wherein the volatile medication is encapsulated with a highly volatile cosolvent.

14. The nasal dilator (10) of any one of claims 1 to 13, wherein multiple volatile medications are provided in a single nasal dilator (10) by mixing microcapsules (82) having only one volatile medication encapsulated therein with microcapsules (82) having a different volatile medication encapsulated therein.

## Patentansprüche

1. Nasal-Dilatator (10) aufweisend
- ein Tragersubstrat (18) aufweisend eine erste Fläche (44) und eine zweite Fläche, wobei die erste Flache (44) mit einer Haftschicht (46) versehen ist und die zweite Fläche mit Mikrokapseln (82) versehen ist, die eine fluchtige Medikation enthalten
**dadurch gekennzeichnet, dass**
- das Trägersubstrat (18) mindestens ein elastisches Federelement (30a,30b) aufweist, und
- eine flussigkeits- oder dampfdurchlässige Schutzschicht (84) über den Mikrokapseln (82) vorgesehen ist.

2. Nasal-Dilatator (10) nach Anspruch 1, wobei die Mikrokapseln (82) eine Bruchfestigkeit aufweisen und in einer Haftkomposition verteilt sind, wobei die Haftkomposition eine Kohasionsfestigkeit und eine erste Bindefestigkeit zur zweiten Flache des Tragers (18) aufweist.

3. Nasal-Dilatator (10) nach Anspruch 1 oder 2, wobei die Mikrokapseln (82) mit der zweiten Fläche durch ein Bindematerial verbunden sind.

4. Nasal-Dilatator (10) nach Anspruch 3, wobei das Bindemittel eine geringe Adhasion hat.

5. Nasal-Dilatator (10) nach einem der Anspruche 1 bis 4, wobei die Bindematrix ausreichend steif bei ungefahr 49°C (120°F) ist, so dass die Mikrokapseln (82) beim Biegen des Nasal-Dilatators (10) bei Anwendung auf der Nase brechen.

6. Nasal-Dilatator (10) nach Anspruch 5, wobei die Bindematrix ausreichend flexibel bei einer Temperatur von ungefähr 60°C (140°F) ist, so dass der Nasal-Dilatator (10) bei einer erhöhten Temperatur ohne nennenswertenMikrokapselbruch hergestellt werden kann.

7. Nasal-Dilatator (10) nach einem der Anspruche 1 bis 6, wobei die Mikrokapseln (82) einen durchschnittlichen Durchmesser von ungefahr 4 bis 200 Mikron aufweisen.

8. Nasal-Dilatator (10) nach einem der Anspruche 1 bis 6, wobei die Mikrokapseln einen durchschnittlichen Durchmesser von ungefähr 300 bis 1000 Mikron aufweisen.

9. Nasal-Dilatator (10) nach einem der Ansprüche 1 bis 8, wobei die flüchtige Medikation aus der Gruppe Kampfer, Eukalyptusol, Pfefferminzöl, Menthol, Methylsalicylat, Bornylazetat, Lavendelol oder Mischungen davon ausgewahlt ist.

10. Nasal-Dilatator (10) nach einem der Anspruche 1 bis 8, wobei die fluchtige Medikation aus der Gruppe bestehend aus transdermalen Abschwellmitteln und Antihistaminika ausgewahlt ist.

11. Nasal-Dilatator (10) nach einem der Ansprüche 1 bis 8, wobei die fluchtige Medikation aus der Gruppe bestehend aus Diphenhydramin, Triprolidin, Ephedrin, Dimethinden, Epinastin, Emedastin und Chloridin ausgewählt ist.

12. Nasal-Dilatator (10) nach einem der Ansprüche 1 bis 8, wobei die fluchtige Medikation aus der Gruppe bestehend aus Aromatherapie-Wirkstoffen und naturlichen Therapie-Wirkstoffen ausgewählt ist.

13. Nasal-Dilatator (10) nach einem der Ansprüche 1 bis 12, wobei die fluchtige Medikation mit einem hoch flüchtigen weitere Lösemittel eingeschlossen ist.

14. Nasal-Dilatator (10) nach einem der Ansprüche 1 bis 13, wobei vielfache fluchtige Medikationen in einem einzigen Nasal-Dilatator (10) bereitgestellt sind, indem Mikrokapseln (82), in denen nur eine flüchtige Medikation eingeschlossen ist, mit Mikrokapseln, in denen verschiedene flüchtige Medikationen eingeschlossen sind, gemischt werden.

## Revendications

1. Dilatateur nasal (10) comprenant :
- un substrat de support (18) comportant une première face (44) et une deuxième face, la première face (44) étant pourvue d'une couche adhésive (46) et la deuxième face étant pourvue de microcapsules (82) contenant un médicament volatil,
**caractérisé en ce que**
- le substrat de support (18) comprend au moins un élément résilient à ressort (30a, 30b), et
- une couche protectrice perméable aux liquides ou à la vapeur (84) est présente sur lesdites microcapsules (82).

2. Dilatateur nasal (10) selon la revendication 1, dans lequel les microcapsules (82) ont une résistance à la rupture et sont dispersées dans une composition adhésive, la composition présentant une force de cohésion et une première force d'adhérence entre elle-même et la deuxième face du support (18).

3. Dilatateur nasal (10) selon la revendication 1 ou 2, dans lequel les microcapsules (82) sont fixées à la deuxième face par un matériau qui est un liant.

4. Dilatateur nasal (10) selon la revendication 3, dans lequel le liant est un encollage envers peu adhésif.

5. Dilatateur nasal (10) selon l'une quelconque des revendications 1 à 4, dans lequel la matrice de liaison est suffisamment rigide à environ 49 °C (120 °F) pour que les microcapsules (82) se rompent au moment de la flexion du dilatateur nasal (10) quand celui-ci est appliqué sur le nez.

6. Dilatateur nasal (10) selon la revendication 5, dans lequel la matrice de liaison est suffisamment flexible à des températures d'environ 60 °C (140 °F) pour que le dilatateur nasal (10) puisse être fabriqué à une température élevée sans rupture substantielle des microcapsules.

7. Dilatateur nasal (10) selon l'une quelconque des revendications 1 à 6, dans lequel les microcapsules (82) ont des diamètres moyens entre environ 4 et 200 microns.

8. Dilatateur nasal (10) selon l'une quelconque des revendications 1 à 6, dans lequel les microcapsules ont des diamètres moyens entre environ 300 et 1 000 microns.

9. Dilatateur nasal (10) selon l'une quelconque des revendications 1 à 8, dans lequel le médicament volatil est sélectionné dans le groupe constitué du camphre, de l'huile d'eucalyptus, de l'huile de menthe poivrée, du menthol, du salicylate de méthyle, de l'acétate de bornyle, de l'huile de lavande, ou de mélanges de ceux-ci.

10. Dilatateur nasal (10) selon l'une quelconque des revendications 1 à 8, dans lequel le médicament volatil est sélectionné dans le groupe constitué de décongestifs transdermiques et d'antihistaminiques.

11. Dilatateur nasal (10) selon l'une quelconque des revendications 1 à 8, dans lequel le médicament volatil est sélectionné dans le groupe constitué de la diphenhydramine, de la triprolidine, de l'éphédrine, du diméthendène, de l'épinastine, de l'émédastine, et de la clonidine.

12. Dilatateur nasal (10) selon l'une quelconque des revendications 1 à 8, dans lequel le médicament volatil est sélectionné dans le groupe constitué d'agents d'aromathérapie et d'agents thérapeutiques naturels.

13. Dilatateur nasal (10) selon l'une quelconque des revendications 1 à 12, dans lequel le médicament volatil est encapsulé dans un co-solvant hautement volatil.

14. Dilatateur nasal (10) selon l'une quelconque des revendications 1 à 13, dans lequel de multiples médicaments volatils sont fournis dans un seul dilatateur nasal (10) par mélange de microcapsules (82) dans lesquelles est encapsulé un seul médicament volatil avec des microcapsules (82) dans lesquelles est encapsulé un autre médicament volatil.
